# EUROPEAN PATENT SPECIFICATION

(11) **EP 4 072 517 B1**
(45) Date of publication and mention of the grant of the patent: **01.04.2026**
(21) Application number: 20820856.1
(22) Date of filing: 06.12.2020
(51) Int. Cl.: A61K 9/00, A61K 47/02, A61K 47/10, A61K 47/18, A61K 47/26, A61K 47/36, A61K 47/38, A61K 31/439, A24B 15/167, A61P 25/34, A61P 25/00

(54) **LIQUID PHARMACEUTICAL COMPOSITION COMPRISING CYTISINE**
FLÜSSIGE PHARMAZEUTISCHE ZUSAMMENSETZUNG, UMFASSEND CYTISIN
COMPOSITION PHARMACEUTIQUE LIQUIDE COMPRENANT DE LA CYTISINE

(30) Priority: 10.12.2019 EP 19461613; 10.12.2019 PL 43215519
(43) Date of publication of application: 19.10.2022
(73) Proprietor: Adamed Pharma S.A., 05-152 Czosnow (PL)
(72) Inventor: KUBIAK, Bartlomiej, 05-101 Nowy Dwor Mazowiecki (PL); RZASA, Joanna, 35-116 Rzeszow (PL); ZARZYCKA, Mariola, 01-961 Warszawa (PL); GARBERA, Kamil, 59-220 Legnica (PL)
(74) Representative: Sitkowska, Jadwiga
(86) International application number: PCT/EP2020/084783
(87) International publication number: WO 2021/115977

(56) References cited:
- EP-A1- 2 815 741
- RU-C1- 2 593 585

## Description

### Field of invention

The present application relates to a liquid pharmaceutical composition comprising cytisine, and the liquid pharmaceutical composition for use in the treatment of tobacco smoking addiction and other forms of nicotine addiction.

### State of art

Tobacco use is the leading cause of preventable morbidity and mortality from various diseases worldwide, killing more than 8 million people a year around the world. Addiction to nicotine is a major barrier to an individual's ability to successfully and permanently stop smoking. Because nicotinic acetylcholine receptors (nAChRs) play a central role in the neuronal mechanism leading to nicotine addiction, they are considered a major target for the development of therapeutic strategies for smoking cessation aids.

In the field of smoking cessation therapy, many treatments utilise nicotine in the form of patches, chewing gum, orally disintegrating tablets and inhalers. These nicotine formulations have some disadvantages, like need of frequent dosing, mouth soreness, dyspepsia and sometimes sleep problems. In addition to nicotine, two partial nicotinic receptor agonists, varenicline and cytisine, are clinically used.

Cytisine has been used as an aid to smoking cessation since the 1960s in a number of Eastern and Central European countries, but has remained relatively unknown to the rest of the world. The growing interest in cytisine as an alternative to varenicline and nicotine has emerged in recent years.

Cytisine is a plant-based alkaloid found in the seeds of the golden rain tree (*Cytisus laburnum*) widely distributed in the Eastern Europe. Chemically, cytisine is (1R-cis)-1,2,3,4,5,6-hexahydro-1,5-methano-8H-pyrido[1,2-a][1,5]diazocin-8-one and shows an affinity towards the specific subunits of nicotine acetylcholine receptors (nAChRs). It acts as a partial agonist at the α4β2 subunit, and very potently binds to the α3β4, α7 nicotinic subunits. The affinity for nicotinic α4β2 receptors is high, Ki = 0.45 µM. Preclinical and clinical data suggest a wide therapeutic index and a favourable safety profile. Pharmacologically, cytisine shows a high degree of similarity to nicotine and is administered in small doses in the form of tablets or capsules, as a substitute for nicotine in mitigation of nicotine cravings in the course of therapies aimed at reduction of negative effects of nicotinism. Recently, solid pharmaceutical forms containing cytisine have become available: Tabex^{®} (Sopharma), Desmoxan (Aflofarm Pharma), Recigar (Adamed Pharma).

Swallowing oral medications in the form of pills, tablets, or capsules presents a persistent challenge for many individuals. Cytisine has a complex dosing regimen that requires a continuous administration for 25 days and taking up to 6 tablets/capsules per day in the first days of the treatment. So, poor compliance with oral medication regimens may occur in patients with general swallowing difficulties (dysphagia) - for example, due to a condition such as a stroke or diabetes.

Moreover, tablets or capsules must always be taken with a sufficient amount of liquids. This is often a problem in places where immediate access to water is limited, especially during travel.

Additionally, a common side effect of cytisine-containing products that affects about 35% of patients is dry mouth, which also enhances unpleasant feeling of lack of freshness in the oral cavity.

Therefore, it would be desirable to provide a more convenient method for oral administration of cytisine, a liquid composition suitable for use in the form of a liquid, a mist, a spray, or an aerosol.

Liquid compositions comprising cytisine are practically unknown. Only Russian application RU2593585 discloses a solution for intranasal application containing cytisine, water, sodium hydrogen phosphate and sodium dihydrogen phosphate as acidity regulators, EDTA, methylparaben (paraben M) and propylparaben (paraben P) as preservatives, sodium chloride as salt, polysorbate as co-solvent and citric acid as antioxidant. The application however discloses three examples exhibiting pH values 7.7 at the concentration of cytisine 1 mg/ml (Example 2), 9.0 at the concentration of cytisine 40 mg/ml (Example 1), and 9.3 at the concentration of cytisine 100 mg/ml (Example 3). No embodiment with pH value of 5.0 and phosphate buffer is disclosed.

### Summary of the invention

The present invention provides a stable liquid composition of cytisine. The composition of the invention is easy to administer and improves patient convenience with discreet and quick application of the composition. Furthermore, the composition is suitable for administration as a mist, a spray or an aerosol.

In the first aspect, the present invention relates to a liquid pharmaceutical composition comprising:
- cytisine,
- at least one inorganic pH-regulating agent selected from the group consisting of inorganic acids, inorganic buffers, and inorganic acid salts,
- water,
wherein the liquid pharmaceutical composition has a pH value between 3.0 and 7.5;
and wherein the liquid composition is free of organic salts, buffers, or acids as pH-regulating agents.

In one embodiment, at least one inorganic pH-regulating agent is selected from the group consisting of hydrogen phosphate, dihydrogen phosphate, hydrogen phosphate/dihydrogen phosphate buffer, hydrochloric acid, phosphoric acid, and mixtures thereof. In one preferable embodiment, at least one inorganic pH-regulating agent is selected from the group consisting of hydrogen phosphate, dihydrogen phosphate, or hydrogen phosphate/dihydrogen phosphate buffer.

In one embodiment, the liquid pharmaceutical composition comprises at least one viscosity agent. In one preferable embodiment, the at least one viscosity agent is present in the amount of 0.01-3% wt. In a more preferable embodiment, the viscosity agent is sodium hyaluronate or carboxymethylcellulose sodium. In the most preferable embodiment, the viscosity agent is sodium hyaluronate.

In one embodiment, the liquid pharmaceutical composition comprises at least one co-solvent. In one preferable embodiment, the liquid pharmaceutical composition comprises at least one co-solvent in the amount of 5-50% wt. In a more preferable embodiment the co-solvent is glycerol or propylene glycol, or mixture thereof.

In one further embodiment the liquid pharmaceutical composition comprises a sweetener selected from the group consisting of a sugar or an artificial sweetener, and mixtures thereof. In the second aspect, the present invention relates to the liquid pharmaceutical composition as defined above for use in the treatment of tobacco smoking addiction and other forms of nicotine addiction. Preferably, the formulation is for administration into the oral cavity of a subject by mist or aerosol.

In one embodiment, the present invention relates, generally, to a liquid aqueous pharmaceutical composition comprising cytisine for oral use in the treatment of tobacco smoking addiction and other forms of nicotine addiction.

### Detailed description of the invention

As mentioned above, not only does the liquid pharmaceutical composition of the invention provide a stable composition comprising cytisine, but also the resulting composition has an acceptable taste.

The present invention relates to a liquid pharmaceutical composition comprising:
- cytisine
- at least one inorganic pH-regulating agent selected from the group consisting of inorganic acids, inorganic buffers, and inorganic acid salts,
- water,
wherein the liquid pharmaceutical composition has a pH value between 3.0 and 7.5; and
wherein the liquid composition is free of organic salts, buffers, or acids as pH-regulating agents.

Preferably, however, the pH value is between 5.0 and 7.0, most preferably, the pH value is between 5.4 and 7.0.

The present inventors have found that the stability of cytisine in liquid compositions depends strongly on the pH value of its solution and on the type of the pH-regulating agent used. Surprisingly, it was found that the liquid pharmaceutical composition of the invention is stable when the liquid pharmaceutical composition has a pH value between 3.0 and 7.5, and when an inorganic pH-regulating agent was used. Furthermore, it turned out that with pH values below 3.0 the taste of the composition was unacceptable. Preferably, however, the liquid pharmaceutical composition has a pH value between 5.0 and 7.0.

Cytisine is usually present in the amount of 0.1-10% wt. Cytisine can be obtained from commercial sources such as Shaanxi The River Pharmaceutical Co., Ltd.

The at least one inorganic pH-regulating agent is used to maintain an approximately constant pH value of the liquid pharmaceutical composition of the invention over time. Preferably, at least one inorganic pH-regulating agent should be present in the amount of 0.5-10% wt.

The at least one inorganic pH-regulating agent is selected from the group consisting of inorganic acids, inorganic buffers, and inorganic acid salts. Of course, the inorganic pH-regulating agent is pharmaceutically acceptable, which means that it is non-toxic and can be used in pharmaceutical compositions.

Cytisine has a strong basic nature, therefore it can be used with an inorganic acid and an inorganic acid salt to provide a buffering system itself. Alternatively, cytisine can be used with an inorganic buffer.

In the present invention, the inorganic acids are typical mineral, pharmaceutically acceptable acids. Preferably, the inorganic acid is selected from hydrochloric acid, hydrobromic acid, sulfuric acid, or phosphoric acid.

In the present invention, the inorganic buffers are one of the following pairs: acid/hydrogen salt of an acid, hydrogen salt/dihydrogen salt, acid/dihydrogen salt of an acid. Preferably, the inorganic buffer is selected from phosphoric acid/dihydrogen phosphate, dihydrogen phosphate/hydrogen phosphate, or phosphoric acid/phosphate. Examples of such buffers are Na₂HPO₄/NaH₂PO₄, H₃PO₄/Na₂HPO₄, H₃PO₄/NaH₂PO₄. The most preferable buffer is Na₂HPO₄/NaH₂PO₄, or its potassium equivalent such as K₂HPO₄/NaH₂PO₄, or Na₂HPO₄/KH₂PO₄, K₂HPO₄/KH₂PO₄.

In the present invention, inorganic acid salt is a salt obtained by a partial neutralisation of inorganic diprotic or polyprotic acids. Preferably, the inorganic diprotic or polyprotic acids are sulfuric acid and phosphoric acid. More preferably, the inorganic acid salt is selected from potassium dihydrogen phosphate, dipotassium hydrogen phosphate, sodium dihydrogen phosphate, sodium hydrogen phosphate, and sodium hydrogen sulfate.

Of course, the at least one inorganic pH-regulating agent is selected and used in such an amount as to assure the desirable pH value of the liquid pharmaceutical composition of the invention. The pH value of the liquid pharmaceutical composition can be measured using a potentiometric pH meter apparatus. A more detailed method for pH value measurement is provided in the Examples.

According to the invention, the liquid pharmaceutical composition of the invention does not comprise any organic salts, buffers, or acids as pH-regulating agents. Preferably, the liquid pharmaceutical composition does not comprise citric acid, or a salt thereof, succinic acid, or a salt thereof, tartaric acid, or a salt thereof, acetic acid, or a salt thereof, as a pH-regulating agent.

The liquid pharmaceutical composition of the invention, as specified above, exhibits high stability, for example, under accelerated conditions (40°C/75% RH), and/or under normal conditions (25°C/65% RH). Preferably, the liquid pharmaceutical composition of the invention, as specified above, exhibits high stability under normal conditions. Preferably, the liquid pharmaceutical composition of the invention exhibits high stability under accelerated conditions.

The liquid pharmaceutical composition of the invention can also contain customary additives that can be used in a pharmaceutical composition suitable for oral administration.

Preferably, the liquid pharmaceutical composition comprises at least one viscosity agent. The viscosity agent provides the liquid pharmaceutical composition with a desired viscosity on the one hand, and on the other hand, it provides an additional moisturising effect to the oral cavity, when it is orally administered as a mist, a spray or an aerosol.

In general, at least one viscosity agent is present in the amount of 0.01-5% wt.

The at least one viscosity agent can be selected from agar, chitosan, carrageenan, tragacanth, xanthan gum, guar gum, alginic acid, propylene glycol alginate, sodium alginate, carboxymethylcellulose calcium, carboxymethylcellulose sodium, colloidal silicon dioxide, ethylcellulose, hydroxyethyl cellulose, hydroxyethylmethyl cellulose, hydroxypropyl cellulose, hypromellose, methylcellulose, gelatin, glycerine, polyvinyl alcohol, povidone, starch, hydroxypropyl. Preferably, however, it is selected from sodium hyaluronate or carboxymethylcellulose sodium.

Surprisingly, it turned out that remarkably good results were achieved with sodium hyaluronate. It showed not only very good moisturising effect in the oral cavity, but also further improved the stability.

Furthermore, the liquid pharmaceutical composition can comprise at least one co-solvent. The at least one co-solvent is contained to aid dissolution of additives and/or to modify taste of the liquid pharmaceutical composition (e.g. in order to provide a sweet taste). The presence of a co-solvent is not mandatory; however, it is preferable. When no co-solvent is added, at least one solubiliser can be added to aid dissolution of further additives as specified below. Such solubilisers can be selected from Poloxamers and Tweens, preferably from Poloxamer 407 and Tween 80.

In general, the at least one co-solvent is present in the amount of 5-50% wt.

The at least one co-solvent can be selected from propylene glycol, polyethylene glycol, glycerine, and ethanol. Preferably, it is selected from propylene glycol or glycerine, or a mixture thereof.

To improve organoleptic properties and mask the taste of the liquid pharmaceutical composition according to the invention, at least one sweetener and/or at least one flavour can be added. The liquid pharmaceutical composition preferably comprises a sweetener.

The sweetener is selected from a sugar, an artificial sweetener, and mixtures thereof. Furthermore, is can be selected from erythritol, fructose, dextrose, saccharin, sorbitol, xylitol, mannitol, maltose, maltitol, maltitol solution, liquid glucose, inulin, isomalt, saccharin sodium, sodium cyclamate, sucralose, sucrose, acesulfame potassium, or aspartame. Preferably, the sweetener is selected from xylitol, mannitol, acesulfame K, aspartame, erythritol, maltitol, or sucrose. Moreover, at least one flavour can be selected from the group comprising mint flavour, tropical flavour, banana flavour, cherry flavour or orange flavour.

To prevent decomposition by microbial growth, a preservative can be added.

The preservative can be selected from benzalkonium chloride, benzalkonium chloride solution, benzethonium chloride, benzoic acid, benzyl alcohol, butylparaben, methylparaben, methylparaben sodium, ethylparaben, propylparaben, propylparaben sodium, cetrimonium bromide, cetylpyridinium chloride, chlorobutanol, chlorocresol, cresol, phenol, potassium benzoate, sodium benzoate, potassium sorbate, sorbic acid, sodium dehydroacetate, sodium propionate or thimerosal.

Preferably, the preservative is selected from methylparaben, propylparaben, and sodium benzoate. According to the invention, the liquid composition is free of organic salts, buffers, or acids as pH-regulating agents.

The liquid pharmaceutical composition of the invention can be simply obtained by dissolving cytisine in water and at least one pH-regulating agent to yield first solution, adding further additives, and completing with water to final volume.

Alternatively, when, for example, a co-solvent is used, the liquid pharmaceutical composition of the invention can be simply obtained by dissolving cytisine in the at least one co-solvent, and adding water and at least one pH-regulating agent to yield first solution, followed by adding further additives, and completing with water to final volume.

Alternatively, when, for example, at least one co-solvent, viscosity agent, preservative, sweetener, and flavour are used, the liquid pharmaceutical composition of the invention can be simply obtained by dissolving all additives in water, followed by dissolving cytisine in such a premix, and completing with water to final volume.

The liquid pharmaceutical composition thus obtained is used as a mouth spray, aerosol or mist for application of the solution directly to the oral cavity and its mucosal lining.

The liquid pharmaceutical composition of the present invention thus obtained can be transferred to a bottle equipped with an atomiser adjusted to dosing 1.5 mg of cytisine. One dose can be simply administered into the oral cavity of a person in the treatment of tobacco smoking addiction and other forms of nicotine addiction, according to the established dosing regimen, as disclosed, for example, in the Recigar^{®} tablet leaflet.

The present invention provides in general a liquid pharmaceutical composition comprising cytisine for oral use in the treatment of tobacco smoking addiction and other forms of nicotine addiction. Such a liquid pharmaceutical composition comprising cytisine for oral use is very beneficial from the point of view of a patient. It can be administered without the need of taking a dose of cytisine (in the form of a tablet or capsule) with a sufficient amount of liquids (water, juice). Furthermore, in a preferable embodiment such as a liquid pharmaceutical composition comprising cytisine also contains a viscosity agent, as defined above. In this embodiment, the viscosity agent provides a moisturising effect in the oral cavity. In the most preferable embodiment, the viscosity agent is hyaluronate such as sodium hyaluronate. This is very important, as a very common side effect is dry mouth, which also enhances unpleasant feeling of lack of freshness in the oral cavity. To make the administration process easy, in general, a liquid aqueous pharmaceutical composition comprising cytisine for oral use is preferably administered as an aerosol, spray or mist using a suitable device such as an atomiser (for example, one manufactured by Aeropump).

### Examples

The present invention is illustrated by the following examples of preparation.

### Method for measuring the pH

The pH is measured according to the European Pharmacopoeia 2.2.3 at room temperature (20-25°C) and atmospheric pressure using a potentiometric pH meter apparatus (such as 913 pH Meter Lab, LL-Unitrode easy Clean 1m) calibrated using pH 2.00, 4.01 and 7.00 buffer solutions.

### Example 1. Preparation of exemplary compositions

Compositions comprising organic salts, buffers, or acids as pH-regulating agents are not according to the invention.

### Composition 1. Preparation of liquid formulation of cytisine at pH 6.0

The quantitative composition of this formulation is disclosed in the table below. Cytisine was dissolved in water together with NaH₂PO₄ dihydrate and Na₂HPO₄ anhydrous to create the first solution. Further, the remaining components xylitol, sodium hyaluronate, mint flavour, paraben M, glycerol anhydrous, paraben M and propylene glycol were added. The solution was stirred until complete dissolution. Finally, water was added to the resulting mixture to the desired volume.

The final pharmaceutical composition is transferred into a HDPE bottle equipped with an atomiser (for example, as manufactured by Aeropump).

| **Component** | **Amount** |
|---|---|
| Cytisine | 0.75% |
| Xylitol | 10.00% |
| Sodium hyaluronate | 0.04% |
| Mint flavour | 0.075% |
| NaH₂PO₄ dihydrate | 3.00% |
| Na₂HPO₄ anhydrous | 0.30% |
| Paraben M | 0.20% |
| Glycerol anhydrous | 21.00% |
| Propylene glycol | 21.00% |
| Water | ad 100% |

### Composition 2. Preparation of liquid formulation of cytisine at pH 7.0

The identical procedure as in Composition 1 was used but the ratios of the ingredients were changed accordingly. NaH₂PO₄ dihydrate was substituted with NaH₂PO₄ monohydrate and xylitol was substituted with mannitol.

| **Component** | **Amount** |
|---|---|
| Cytisine | 1.50% |
| Mannitol | 5.00% |
| Sodium hyaluronate | 0.10% |
| Tropical flavour | 0.075% |
| NaH₂PO₄ monohydrate | 2.00% |
| Na₂HPO₄ anhydrous | 0.30% |
| Paraben M | 0.10% |
| Glycerol anhydrous | 11.00% |
| Propylene glycol | 11.00% |
| Water | ad 100% |

### Composition 3. Preparation of liquid formulation of cytisine at pH 6.8

Cytisine was dissolved in water together with NaH₂PO₄ to create the first solution. Further, xylitol, sodium carboxymethylcellulose, glycerol anhydrous and propylene glycol were added. The solution was stirred until complete dissolution. Finally, water was added to the resulting mixture to the desired volume.

| **Component** | **Amount** |
|---|---|
| Cytisine | 3.00% |
| Xylitol | 5.00% |
| Sodium carboxymethylcellulose | 0.50% |
| NaH₂PO₄ dihydrate | 4.00% |
| Glycerol 85% wt. | 11.00% |
| Propylene glycol | 25.00% |
| Water | ad 100% |

### Composition 4. Preparation of liquid formulation of cytisine at pH 5.9

Cytisine was dissolved in water together with potassium phosphate monobasic, sodium chloride and methylcellulose to create the first solution. Further, tween 80, glycerol anhydrous and paraben P were added. The solution was stirred until complete dissolution. Finally, water was added to the resulting mixture to the desired volume.

| **Component** | **Amount** |
|---|---|
| Cytisine | 1.00% |
| Potassium phosphate monobasic | 4.00% |
| Methylcellulose | 1.0% |
| Glycerol anhydrous | 11.00% |
| Tween 80 | 0.05% |
| Sodium chloride | 0.5% |
| Paraben P | 0.05% |
| Water | ad 100% |

### Composition 5. Preparation of liquid formulation of cytisine at pH 3

Cytisine was dissolved in water together with xylitol, sodium hyaluronate and sodium benzoate to create the first solution. Further, banana flavour and glycerol anhydrous were added. The solution was stirred until complete dissolution. Then hydrochloric acid was added to obtain pH 3.0. Finally, water was added to the resulting mixture to the desired volume.

| **Component** | **Amount** |
|---|---|
| Cytisine | 0.50% |
| Xylitol | 15.00% |
| Sodium hyaluronate | 0.10% |
| Banana flavour | 0.10% |
| Sodium benzoate | 0.10% |
| Glycerol anhydrous | 11.00% |
| Hydrochloric acid | q.s. to obtain pH 3.0 |
| Water | ad 100% |

### Composition 6. Preparation of liquid formulation of cytisine at pH 4.5

Cytisine was dissolved in water together with xylitol, sodium hyaluronate and sodium benzoate to create the first solution. Further, banana flavour and glycerol anhydrous were added. The solution was stirred until complete dissolution. Then diluted phosphoric acid was added to obtain pH 4.5. Finally, water was added to the resulting mixture to the desired volume.

| **Component** | **Amount** |
|---|---|
| Cytisine | 0.50% |
| Xylitol | 15.00% |
| Sodium hyaluronate | 0.50% |
| Banana flavour | 0.10% |
| Sodium benzoate | 0.10% |
| Glycerol anhydrous | 11.00% |
| Phosphoric acid diluted | q.s. to obtain pH 4.5 |
| Water | ad 100% |

### Composition 7. Preparation of liquid formulation of cytisine at pH 7.5

Cytisine was dissolved in water together with xylitol, sodium hyaluronate and NaH₂PO₄ dihydrate to create the first solution. Further, mint flavour, propylene glycol with paraben M and glycerol anhydrous were added. The solution was stirred until complete dissolution. Finally, water was added to the resulting mixture to the desired volume.

| **Component** | **Amount** |
|---|---|
| Cytisine | 0.70% |
| Xylitol | 7.00% |
| Sodium hyaluronate | 0.05% |
| Mint flavour | 0.10% |
| NaH₂PO₄ dihydrate | 0.55% |
| Glycerol anhydrous | 20.00% |
| Propylene glycol | 20.00% |
| Paraben P | 0.06% |
| Water | ad 100% |

### Composition 8. Preparation of liquid formulation of cytisine at pH 6.9

Cytisine was dissolved in water together with acesulfame K, phosphoric acid and NaH₂PO₄ dihydrate to create the first solution. Further, propylene glycol with paraben M, glycerol anhydrous and mint flavour were added. The solution was stirred until complete dissolution. Finally, water was added to the resulting mixture to the desired volume.

| **Component** | **Amount** |
|---|---|
| Cytisine | 1.00% |
| Acesulfame K | 0.10% |
| Mint flavour | 0.05% |
| NaH₂PO₄ dihydrate | 1.00% |
| Phosphoric acid | 0.10% |
| Glycerol anhydrous | 11.00% |
| Propylene glycol | 11.00% |
| Paraben M | 0.10% |
| Water | ad 100% |

### Composition 9. Preparation of liquid formulation of cytisine at pH 6.9

Cytisine was dissolved in water together with aspartam, NaH₂PO₄ dihydrate and hydroxypropylmethylcellulose to create the first solution. Further, orange flavour and propylene glycol were added. The solution was stirred until complete dissolution. Finally, water was added to the resulting mixture to the desired volume.

| **Component** | **Amount** |
|---|---|
| Cytisine | 0.30% |
| Aspartam | 0.20% |
| Hydroxypropylmethylcellulose | 0.50% |
| Orange flavour | 0.05% |
| NaH₂PO₄ dihydrate | 3.00% |
| Propylene glycol | 15.00% |
| Water | ad 100% |

### Composition 10. Preparation of liquid formulation of cytisine at pH 5.9

Propylene glycol and paraben M was dissolved in some water together with erythritol, hydroxyethylcellulose and NaH₂PO₄ dihydrate to create the first solution. Further, cytisine and cherry flavour were added. The solution was stirred until complete dissolution. Finally, water was added to the resulting mixture to the desired volume.

| **Component** | **Amount** |
|---|---|
| Cytisine | 0.50% |
| Erythritol | 1.00% |
| Hydroxyethylcellulose | 0.50% |
| Cherry flavour | 0.05% |
| NaH₂PO₄ dihydrate | 3.00% |
| Paraben M | 0.10% |
| Glycol propylene | 5.00% |
| Water | ad 100% |

### Composition 11. Preparation of liquid formulation of cytisine at pH 5.0

Cytisine was dissolved in water together with maltitol liquid, NaH₂PO₄ dihydrate and carboxymethylcellulose sodium to create the first solution Further, cherry flavour, propylene glycol with paraben M and P were added. The solution was stirred until complete dissolution. Finally, water was added to the resulting mixture to the desired volume.

| **Component** | **Amount** |
|---|---|
| Cytisine | 0.50% |
| Maltitol liquid | 10.00% |
| Carboxymethylcellulose sodium | 0.20% |
| Cherry flavour | 0.05% |
| NaH₂PO₄ dihydrate | 9.00% |
| Paraben M | 0.10% |
| Paraben P | 0.05% |
| Propylene glycol | 10.00% |
| Water | ad 100% |

### Composition 12. Preparation of liquid formulation of cytisine at pH 5.4

Tween 80 and paraben P were dissolved in some water together with sucrose, poloxamer and NaH₂PO₄ dihydrate to create the first solution. Further, cytisine was added. The solution was stirred until complete dissolution. Finally, water was added to the resulting mixture to the desired volume.

| **Component** | **Amount** |
|---|---|
| Cytisine | 0.50% |
| Sucrose | 5.00% |
| Poloxamer 407 | 0.50% |
| NaH₂PO₄ dihydrate | 6.00% |
| Paraben P | 0.05% |
| Tween 80 | 0.05% |
| Water | ad 100% |

### Composition 13. Preparation of liquid formulation of cytisine at pH 6.1

Propylene glycol and paraben M was dissolved in some water together with xylitol with sodium hyaluronate and NaH₂PO₄ dihydrate to create the first solution. Further, cytisine, glycerol anhydrous and mint flavour were added. The solution was stirred until complete dissolution. Finally, water was added to the resulting mixture to the desired volume.

| **Component** | **Amount** |
|---|---|
| Cytisine | 0.700% |
| Xylitol | 7.00% |
| Sodium hyaluronate | 0.10% |
| Mint flavour | 0.15% |
| NaH₂PO₄ dihydrate | 3.00% |
| Paraben M | 0.10% |
| Glycerol anhydrous | 21.00% |
| Propylene glycol | 15.00% |
| Water | ad 100% |
| | pH = 6.1 |

### Example 2.

### Stability of the liquid pharmaceutical composition of the invention

The liquid pharmaceutical compositions of the invention were stored in a climatic chamber at 40°C/75% Relative Humidity (RH) (accelerated conditions) and 25°C/65% RH (normal conditions), and if necessary at 60°C/ 75% RH, and 30°C/ 65% RH , for a specified time. Samples taken at the end of the storage, or at an intermediate time point, were subjected to HPLC analysis to determine known impurities (FO - N-formylcytisine, MO - N-methylcytisine) and unknown impurities. All pharmaceutical compositions of the invention tested were stable and fulfilled the specification for pharmaceutical drug products with cytisine (FO impurity ≤0.5%, ME impurity ≤0.5%, max. single unknown impurity ≤0.2%, total impurities ≤1.0%).

A stability comparison of the liquid composition from Example 13 with the representative liquid formulation from publication of Russian patent RU2593585 (example 2) is presented in the Table below.

| **Composition 13** | | **RU2593585 (example 2)*** | |
|---|---|---|---|
| **Ingredient** | **Amount [%]** | **Ingredient** | **Amount [mg]** |
| Cytisine | 0.70 | Cytisine | 1000.0 |
| Xylitol | 7.00 | NaH₂PO₄ dihydrate | 250.0 |
| Sodium hyaluronate | 0.10 | Na₂HPO₄ anhydrous | 200.0 |
| Mint flavour | 0.15 | Paraben M | 300.0 |
| NaH₂PO₄ dihydrate | 3.00 | Paraben P | 7.0 |
| Paraben M | 0.10 | EDTA | 10.0 |
| Glycerol anhydrous | 21.00 | Citric acid | 10.0 |
| Propylene glycol | 15.00 | Sodium chloride | 8400.0 |
| - | - | Polysorbate | 500.0 |
| Water | ad 100% | Water | Add to obtain 1 litre of solution |
| **pH** | **6.1** | **pH** | **7.7** |

| Stability results | | | |
|---|---|---|---|
| 40°C/ 75% RH, 1 month | | | |
| FO | <0.03% | FO | <0.03% |
| ME | <0.03% | ME | <0.03% |
| **Total impurities**** | **0.077%** | **Total impurities**** | **0.16%** |

| 60°C/ 75% RH, 1 week | | | |
|---|---|---|---|
| FO | 0.045% | FO | <0.03% |
| ME | 0.041% | ME | <0.03% |
| **Total impurities **** | **0.15%** | **Total impurities**** | **0.22%** |

| 30°C/ 65% RH, 1 month | | | |
|---|---|---|---|
| FO | <0.03% | FO | <0.03% |
| ME | <0.03% | ME | <0.03% |
| **Total impurities **** | **0.04%** | **Total impurities**** | **0.11%** |
| * Example 2 from RU2593585 was prepared in analogy to Composition 4 | | | |
| ** Total impurities include known impurities, FO, ME, as well as all unknown impurities | | | |

As can be seen, the chemical stability profile of the compositions of the invention is significantly better than the representative example 2 from Russian application RU2593585 irrespective of storage conditions. The other two examples from RU2593585 (example 1 and example 3 having pH values 9.0 and 9.3, respectively) exhibited much higher pH values, and turned out to have a lower stability (data not shown).

All formulations tested had better stability than example 2 from RU2593585.

### Example 3.

### Comparison of stability of the liquid pharmaceutical composition of the invention with and without hyaluronate.

Two additional pharmaceutical compositions (Composition 14 and Composition 15) were prepared, analogously to Composition 1.

| **Ingredient** | **Composition 14** | **Composition 15** |
|---|---|---|
| Cytisine | 0.73% | 0.73% |
| Xylitol | 10.00% | 10.00% |
| Sodium hyaluronate | 0.04% | - |
| Mint flavour | 0.075% | 0.075% |
| NaH₂PO₄ dihydrate | 4.00% | 4.00% |
| Na₂HPO₄ anhydrous | 0.30% | 0.30% |
| Paraben M | 0.20% | 0.20% |
| Glycerol anhydrous | 21.00% | 21.00% |
| Propylene glycol | 21.00% | 21.00% |
| Water | Ad 100.00% | Ad 100.00% |
| | **pH=6.1** | **pH=6.1** |

| 25°C/ 60% RH, 6 months | | |
|---|---|---|
| FO | <0.03% | <0.03% |
| ME | <0.03% | <0.03% |
| **Total impurities** | **0.065%** | **0.122%** |

| 40°C/ 75% RH, 3 months | | |
|---|---|---|
| FO | 0.064% | 0.072% |
| ME | <0.03% | <0.03% |
| **Total impurities** | **0.33%** | **0.42%** |

As can be seen, the chemical stability profile of the composition with Sodium hyaluronate (Composition 14) is better than the composition without Sodium hyaluronate (Composition 15) irrespective of storage conditions. Both formulations fulfil, however, the specification criteria, but the use of Sodium hyaluronate is particularly preferable in the composition as it improves chemical stability of the drug product, and additionally increases the moisturising effect in the oral cavity.

### Example 4.

### Comparison of stability of the liquid pharmaceutical composition of the invention and two comparative compositions comprising organic buffer and organic-inorganic buffer.

A composition of the invention (Composition 16) and two comparative compositions (Comparative composition 1, Comparative composition 2) were prepared by simple mixing the given amounts of ingredients as presented in the table below. The compositions thus obtained were kept at 60°C for one week. Chemical purity results are also given below.

| | **Composition 16** | **Comparative composition** 1 | **Comparative composition 2** |
|---|---|---|---|
| Cytisine | 0,2 g | 0,2 g | 0,2 g |
| pH-regulating agent | NaH₂PO₄ dihydrate 1,2 g | sodium citrate dihydrate 0,8 g | sodium succinate 2,6 g |
| pH-regulating agent | Na₂HPO₄ 0,4 g | NaH₂PO₄ dihydrate 0,8 g | succinic acid 0,2 g |
| Water | 20 ml | 20 ml | 20 ml |
| pH | **6,0** | **6,0** | **6,0** |

| 60°C, 1 week | | | |
|---|---|---|---|
| FO | - | <0,03% | - |
| ME | - | - | - |
| **Total impurities** | **0,08%** | **1,08%** | **0,29%** |

The cited results show that the buffer based on inorganic pH-regulating agents provided the best stability when heated at 60°C for one week.

Similarly, two further comparative liquid pharmaceutical compositions (Comparative composition 3 and Comparative composition 4) at pH 4,5 comprising customary additives were prepared. Their stability were also found to be lower than the stability for Composition 6 according to the invention. For example, in the case of Comparative Composition 4 containing a citrate buffer, after keeping at 60°C, 75% RH, the total impurities reached more than 7 wt. % after 1 month.

| Comparative composition 3 | | Comparative composition 4 | |
|---|---|---|---|
| Cytisine | 1,00% | Cytisine | 0,150% |
| Xylitol | 10,00% | Xylitol | 10,00% |
| Sodium hyaluronate | 0,10% | Sodium carboxymethylcellulose | 0,50% |
| **Tartaric acid** | 1,20% | **Citric acid hydrate** | 2,00% |

| **Sodium dihydrogen phosphate** | 2,00% | **Sodium citrate dihydrate** | 1,25% |
|---|---|---|---|
| Menthol flavour liquid | 0,25% | Menthol flavour liquid | 0,15% |
| Paraben M | 0,30% | Paraben M | 0,30% |
| Propylene glycol | 21,00% | Propylene glycol | 21,00% |
| - | - | Glycerol | 21,00% |
| Water | Ad 100% | Water | Ad 100% |
| **pH** | **4,5** | **pH** | **4,5** |

## Claims

1. A liquid pharmaceutical composition comprising:
- cytisine,
- at least one inorganic pH-regulating agent selected from the group consisting of inorganic acids, inorganic buffers, and inorganic acid salts, and
- water, wherein the liquid pharmaceutical composition has a pH value between 3.0 and 7.5; and
wherein the liquid composition is free of organic salts, buffers, or acids as pH-regulating agents.

2. The liquid pharmaceutical composition according to claim 1 wherein the at least one inorganic pH-regulating agent is selected from the group consisting of hydrogen phosphate, dihydrogen phosphate, hydrogen phosphate/dihydrogen phosphate buffer, hydrochloric acid, phosphoric acid, and mixtures thereof.

3. The liquid pharmaceutical composition according to claim 2 wherein the at least one inorganic pH-regulating agent is selected from the group consisting of hydrogen phosphate, dihydrogen phosphate, and hydrogen phosphate/dihydrogen phosphate buffer.

4. The liquid pharmaceutical composition according to any one of claims 1-3 wherein the liquid pharmaceutical composition comprises at least one viscosity agent.

5. The liquid pharmaceutical composition according to claim 4 wherein the liquid pharmaceutical composition comprises the at least one viscosity agent in the amount of 0.01-3% wt.

6. The liquid pharmaceutical composition according to claim 5 wherein the at least one viscosity agent is sodium hyaluronate or carboxymethylcellulose sodium.

7. The liquid pharmaceutical composition according to claim 6 wherein the at least one viscosity agent is sodium hyaluronate.

8. The liquid pharmaceutical composition according to any one of claims 1-7 wherein the liquid pharmaceutical composition comprises at least one co-solvent.

9. The liquid pharmaceutical composition according to claim 8 wherein the liquid pharmaceutical composition comprises the at least one co-solvent in the amount of 5-50% wt.

10. The liquid pharmaceutical composition according to claim 9 wherein the at least one co-solvent is selected from the group consisting of glycerol, propylene glycol, and mixtures thereof.

11. The liquid pharmaceutical composition according to any one of claims 1-10 wherein the liquid pharmaceutical composition comprises a sweetener selected from the group consisting of a sugar, an artificial sweetener, and mixtures thereof.

12. The liquid pharmaceutical composition according to claim 11 wherein the sweetener is selected from the group consisting of xylitol, mannitol, maltitol, sucrose, acesulfame K, aspartame, and mixtures thereof.

13. The liquid pharmaceutical composition according to any one of claims 1-12 for use in the treatment of tobacco smoking addiction and other forms of nicotine addiction.

14. A liquid pharmaceutical composition for use according to claim 13 wherein the liquid pharmaceutical composition is for administration into the oral cavity of a subject by mist or aerosol.

## Patentansprüche

1. Eine flüssige pharmazeutische Zusammensetzung, umfassend:
- Cytisin,
- mindestens ein anorganisches pH-Regulierungsmittel, ausgewählt aus der Gruppe bestehend aus anorganischen Säuren, anorganischen Puffern und anorganischen Säuresalzen,
- Wasser, wobei die flüssige pharmazeutische Zusammensetzung einen pH-Wert zwischen 3,0 und 7,5 aufweist,
und wobei die flüssige pharmazeutische Zusammensetzung von organischen Salzen, Puffern oder Säuren als pH-Regulierungsmittel frei ist.

2. Die flüssige pharmazeutische Zusammensetzung nach Anspruch 1, wobei das mindestens ein anorganisches pH-Regulierungsmittel aus der Gruppe bestehend aus Hydrogenphosphat, Dihydrogenphosphat, Hydrogenphosphat/Dihydrogenphosphat-Puffer, Salzsäure, Phosphorsäure und Mischungen davon ausgewählt ist.

3. Die flüssige pharmazeutische Zusammensetzung nach Anspruch 2, wobei das mindestens ein anorganisches pH-Regulierungsmittel aus der Gruppe bestehend aus Hydrogenphosphat, Dihydrogenphosphat und Hydrogenphosphat/Dihydrogenphosphat-Puffer ausgewählt ist.

4. Die flüssige pharmazeutische Zusammensetzung nach einem der Ansprüche 1-3, wobei die flüssige pharmazeutische Zusammensetzung mindestens ein Viskositätsmittel enthält.

5. Die flüssige pharmazeutische Zusammensetzung nach Anspruch 4, wobei die flüssige pharmazeutische Zusammensetzung mindestens ein Viskositätsmittel in der Menge von 0,01-3 Gew.-% enthält.

6. Die flüssige pharmazeutische Zusammensetzung nach Anspruch 5, wobei das mindestens ein Viskositätsmittel Natriumhyaluronat oder Carboxymethylcellulose-Natrium ist.

7. Die flüssige pharmazeutische Zusammensetzung nach Anspruch 6, wobei das mindestens ein Viskositätsmittel Natriumhyaluronat ist.

8. Die flüssige pharmazeutische Zusammensetzung nach einem der Ansprüche 1 bis 7, wobei die flüssige pharmazeutische Zusammensetzung mindestens ein Co-Lösungsmittel umfasst.

9. Die flüssige pharmazeutische Zusammensetzung nach Anspruch 8, wobei die flüssige pharmazeutische Zusammensetzung mindestens ein Co-Lösungsmittel in der Menge von 5-50 Gew.-% enthält.

10. Die flüssige pharmazeutische Zusammensetzung nach Anspruch 9, wobei das mindestens ein Co-Lösungsmittel aus der Gruppe bestehend aus Glycerin, Propylenglykol und Mischungen davon ausgewählt ist.

11. Die flüssige pharmazeutische Zusammensetzung nach einem der Ansprüche 1-10, wobei die flüssige pharmazeutische Zusammensetzung einen Süßstoff umfasst, die aus der Gruppe bestehend aus Zucker, einem künstlichen Süßstoff, und Mischungen davon ausgewählt ist.

12. Die flüssige pharmazeutische Zusammensetzung nach Anspruch 11, wobei der das Zucker aus der Gruppe bestehend aus Xylit, Mannit, Maltit, Saccharose, Acesulfam K, Aspartam oder Mischungen davon ausgewählt ist.

13. Die flüssige pharmazeutische Zusammensetzung nach einem der Ansprüche 1-12 zur Anwendung bei der Behandlung der Tabakabhängigkeit und anderer Formen der Nikotinabhängigkeit.

14. Eine flüssige pharmazeutische Zusammensetzung zur Verwendung nach Anspruch 13, wobei die flüssige pharmazeutische Zusammensetzung zur Verabreichung in die Mundhöhle eines Patienten mittels Nebel oder Aerosol bestimmt ist.

## Revendications

1. Une composition pharmaceutique liquide comprenant :
- cytisine,
- au moins un agent régulateur de pH inorganique choisi parmi le groupe constitué des acides inorganiques, des tampons inorganiques et des sels d'acides inorganiques,
- eau, dans laquelle la composition pharmaceutique liquide a une valeur de pH comprise entre 3,0 et 7,5,
et dans laquelle la composition pharmaceutique est exempte des sels, des tampons, ou acides organiques comme agents régulateurs de pH.

2. La composition pharmaceutique liquide selon la revendication 1, dans laquelle au moins un agent régulateur de pH inorganique est choisi parmi le groupe constitué de l'hydrogénophosphate, du dihydrogénophosphate, du tampon hydrogénophosphate/-dihydrogénophosphate, de l'acide chlorhydrique, de l'acide phosphorique et de leurs mélanges.

3. La composition pharmaceutique liquide selon la revendication 2, dans laquelle au moins un agent régulateur de pH inorganique est choisi parmi le groupe constitué d'hydrogénophosphate, de dihydrogénophosphate et de tampon hydrogéno-phosphate/dihydrogénophosphate.

4. La composition pharmaceutique liquide selon l'une quelconque des revendications 1-3, dans laquelle la composition pharmaceutique liquide comprend au moins un agent de viscosité.

5. La composition pharmaceutique selon la revendication 4, dans laquelle la composition pharmaceutique liquide comprend au moins un agent de viscosité en quantité de 0,01 à 3 % en poids.

6. La composition pharmaceutique selon la revendication 5, dans laquelle au moins un agent de viscosité est l'hyaluronate de sodium ou la carboxyméthylcellulose sodique.

7. La composition pharmaceutique selon la revendication 6, dans laquelle au moins un agent de viscosité est l'hyaluronate de sodium.

8. La composition pharmaceutique liquide selon l'une quelconque des revendications 1-7, dans laquelle la composition pharmaceutique liquide comprend au moins un co-solvant.

9. La composition pharmaceutique selon la revendication 8, dans laquelle la composition pharmaceutique liquide comprend au moins un co-solvant en quantité de 5 à 50 % en poids.

10. La composition pharmaceutique selon la revendication 9, dans laquelle au moins un co-solvant est choisi parmi le groupe constitué du glycerol, propylène glycol, est des leurs mélanges.

11. La composition pharmaceutique selon l'une quelconque des revendications 1-10, dans laquelle la composition pharmaceutique liquide comprend un édulcorant choisi parmi le groupe constitué du sucre, du édulcorant artificiel, et des leurs mélanges.

12. La composition pharmaceutique selon la revendication 11, dans laquelle l'édulcorant est choisi parmi le groupe constitué du xylitol, du mannitol, du maltitol, du saccharose, l'acésulfame K, de l'aspartame est des leurs mélanges.

13. La composition pharmaceutique selon l'une quelconque des revendications 1-10 pour le traitement de la dépendance au tabac et d'autres formes de dépendance à la nicotine.

14. La composition pharmaceutique pour utilisation dans le traitement selon la revendication 13, dans laquelle la composition pharmaceutique liquide est destinée à être administrée dans la cavité buccale d'un sujet par brumisation ou aérosol.
